# EUROPEAN PATENT SPECIFICATION

(11) **EP 4 204 897 B1**
(45) Date of publication and mention of the grant of the patent: **14.08.2024**
(21) Application number: 20797358.7
(22) Date of filing: 29.09.2020
(51) Int. Cl.: G02C 5/04, G02C 13/00, G02B 27/01

(54) **FITTING MECHANISMS FOR EYEWEAR WITH VISIBLE DISPLAYS AND ACCOMODATION OF WEARER ANATOMY**
ANPASSUNGSMECHANISMEN FÜR BRILLEN MIT SICHTBAREN ANZEIGEN UND AUFNAHME DER TRÄGERANATOMIE
MÉCANISMES D'AJUSTEMENT POUR LUNETTES À ÉCRANS VISIBLES ET ADAPTATION À L'ANATOMIE DE L'UTILISATEUR

(43) Date of publication of application: 05.07.2023
(73) Proprietor: Google LLC, Mountain View, CA 94043 (US)
(72) Inventor: OLWAL, Alex, Mountain View, California 94043 (US); VOTINTCEV, Dmitrii, Mountain View, California 94043 (US)
(74) Representative: Maikowski & Ninnemann Patentanwälte Partnerschaft mbB
(86) International application number: PCT/US2020/070593
(87) International publication number: WO 2022/071975

(56) References cited:
- EP-A1- 3 570 092
- WO-A1-2016/077696
- WO-A1-2016/166270
- WO-A1-2019/198979
- CN-A- 110 927 988
- DE-A1- 102017 105 366
- US-A1- 2013 318 776
- US-A1- 2015 103 306
- US-B1- 6 698 882

## Description

### FIELD

This disclosure relates to eyeglasses having a capability of displaying information electronically as well as allowing a user to see through.

### BACKGROUND

Eyewear (i.e., glasses, also known as eyeglasses or spectacles) are vision aids, consisting of glass or hard plastic lenses mounted in a frame that holds them in front of a person's eyes, typically utilizing a nose bridge over the nose, and legs (known as temples or temple pieces) which rest over the ears.

Smart eyewear are glasses (or smart glasses) that add information alongside what the wearer sees through the glasses. Superimposing information (e.g., digital images) onto a field of view may be achieved through smart optics such as an optical head-mounted display (OHMD), or embedded wireless glasses with transparent heads-up display (HUD), or augmented reality (AR) overlay. Modern smart eyewear are effectively wearable computers which can run self-contained mobile apps. Some may be handsfree and can communicate with the Internet via natural language voice commands, while others use touch buttons.

Consideration is now being given to simplifying assembly and custom fitting of smart eyewear to individual wearers. Comparable eyewear are disclosed in US 2015/1033306 A1, WO 2016/077696 A1, US 6,698,882 B1, EP 3 570092 A1, WO 2016/166270 A1, CN 110 927 988 A, US 2013/318776 A1 and DE 10 2017 105366 A1.

### SUMMARY

The proposed solution relates to a method as stated in claim 1 of the accompanying claim set. In a general aspect, an eyewear frame includes two half-frames. An adjustable nose bridge is disposed between the two half-frames. The two half-frames are adapted to hold a pair of see-through lenses. A virtual display associated with at least one of the two half-frames, the virtual display having an associated eye box for full viewing of the virtual display. The adjustable nose bridge includes one or more adjustable bridge-to-frame fastener arrangements adapted to provide independent adjustments of one or more geometrical parameters of the eyeglasses frame for aligning a position of the eye box relative to the eyeglasses frame for full viewing of the virtual display, wherein the independent adjustment of each geometrical parameter of the eyeglasses frame is independent of adjustments of the other geometrical parameter of the eyeglasses frame. The independent adjustments of the geometrical parameters are carried out one-by-one.

### BRIEF DESCRIPTION OF THE DRAWINGS

Example embodiments will become more fully understood from the detailed description herein and the accompanying drawings, wherein like elements are represented by like reference numerals, which are given by way of illustration only and thus are not limiting of the example embodiments.
FIG. 1A, FIG. 1B, and FIG. 1C illustrate example interchangeable or locally adjustable components used for assembling smart eyewear.
FIG. 2A and FIG. 2B illustrate example eyeglasses frames with different interpupillary distance settings.
FIGS. 3A and 3B illustrate a front view and a side view of an example nose pad assembly.
FIG. 3C illustrates nose pad assemblies attached to a right half-frame and a left half-frame in a glasses frame.
FIGS. 4A and 4B illustrates perspective views of frames with nose pads attached to right half-frames and left half-frames.
FIG. 5A illustrates a top view of adjustable nose bridge with no wrap angle.
FIG. 5B illustrates a top view of adjustable nose bridge with a wrap angle.
FIG. 6A illustrates a perspective top view of an eyeglasses frame interconnected by an adjustable nose bridge with substantially no wrap angle.
FIG. 6B illustrates a perspective top view of an eyeglasses frame interconnected by an adjustable nose bridge that is bent to wrap the frame with a wrap angle.
FIG. 6C illustrates a perspective view of an eyeglasses frame in which right and left lens have substantially no cyclo-rotation.
FIG. 6D illustrates a perspective view of an eyeglasses frame in which right and left lens are cyclo- rotated.
FIGS. 7A and 7B illustrate a fitting procedure for aligning optics of eyeglasses frames to a person's face and eye geometry using an adjustable nose bridge.
FIGS. 8A, 8B and 8C illustrates example methods for aligning optics of an eyeglasses frame to a person's face and eye geometry.
FIG. 9A illustrates a cross sectional view of an example nose bridge having with a fixed geometry.
FIG. 9B illustrate the nose bridge of FIG. 9A fastened to a right half-frame and left half-frame.
FIG. 9C illustrates a cross sectional view of an example nose bridge having with a fixed geometry including a predetermined wrap angle.
FIG. 9D illustrates the nose bridge of FIG. 9C fastened to a right half-frame and left half-frame.
FIG. 10 illustrates an example eyeglasses component kit for assembling eyeglasses fitted to a person.
FIG. 11A illustrates another an example nose bridge having with a fixed geometry.
FIG. 11B illustrates an example cosmetic cover for a nose bridge.
FIG. 11C illustrates a receiving hole that can be used to fasten a nose bridge to a half frame.
FIG. 11D illustrates attachment of a nose bridge to a right half-frame.
FIG. 11E illustrates an eyewear in which nose bridge couples a right half-frame and a left half-frame.
FIG. 12 illustrates a method for capturing anthropometric or biometric characteristics of a person.

It should be noted that these FIGS. are intended to illustrate the general characteristics of methods, structure and/or materials utilized in certain example embodiments and to supplement the written description provided below. These drawings are not, however, to scale and may not precisely reflect the precise structural or performance characteristics of any given embodiment, and should not be interpreted as defining or limiting the range of values or properties encompassed by example embodiments. For example, the relative thicknesses and positioning of components of the described eyeglasses may be reduced or exaggerated for clarity. The use of similar or identical reference numbers in the various drawings is intended to indicate the presence of a similar or identical element or feature.

### DETAILED DESCRIPTION OF THE EMBODIMENTS

Smart glasses can have virtual image displays (hereinafter "virtual display") that add information to what a wearer sees through the glasses. A virtual display may, for example, be an in-lens micro display, or a display projected on a lens surface, or a display projected on a plane of a lens-less frame, etc. Traditional smart glasses do not allow the wearer to adjust the position of the virtual display independently from an overall anatomical fitting of the smart glasses on the wearer. In many instances, smart glasses that have virtual displays (e.g., in-lens micro displays, etc.) are available only in one-size-fits-all versions without regard to variations in the wearers' anatomies. Yet other smart glasses that have virtual displays are custom fabricated at a remote factory for each individual wearer based on previously obtained anthropometric measurements (e.g., by a custom 3D-image scanning) of the individual wearer.

Disclosed herein is eyewear (e.g., smart eyewear) that can be custom fit to a wearer during on-site assembly of the eyewear on the wearer's face, in accordance with the principles of the present disclosure.

In an example implementation, the smart eyewear may be assembled from components that are interchangeable or are locally adjustable on-site. For example, the smart eyewear may be assembled by joining two half-frames using wires, bands, and/or other joining means to form a spectacle frame (hereinafter "frame" , or "eyeglasses frame") and hold a pair of see-through lenses in front of a person's eyes. The joining means can include a locally adjustable nose bridge. A virtual display may be overlaid on, or embedded in, at least one of the pair of see-through lenses.

The eyeglasses frame may utilize the adjustable nose bridge over the person's nose, and utilize temple pieces that rest over the person's ears, to hold the pair of see-through lenses (including the virtual display) in position in front of the person's eyes.

The temple pieces attached to eyeglasses frame may include electronics to prepare and send data (e.g., captions, images) for display on the virtual display. The virtual display may have an associated eye box for full viewing of the virtual display.

The adjustable nose bridge may include mechanisms that can provide independent local adjustments of geometrical parameters of the eyeglasses frame (e.g., interpupillary distance, wrap angle, cyclo-rotation, etc.) for aligning optics of the eyeglasses to the person's face and eye geometry. Aligning optics of the eyeglasses may, for example, include aligning a position of the eye box relative to the eyeglasses frame for full viewing of the virtual display. The example adjustable nose bridges described herein enable at least three degrees of freedom (DOF) of independent adjustments (e.g., interpupillary distance, wrap angle, cyclo-rotation, etc.) for aligning the position of the eye box relative to the eyeglasses frame for full viewing of the virtual display.

The interchangeable or locally adjustable components used for assembling smart eyewear may also include nose pads that can be attached to the two half-frames. The nose pads may be locally adjustable to make a comfortable fit to the person's nose and to set a height of the eyeglasses frame on the person's nose for aligning optics of the eyeglasses to the person's face and eye geometry.

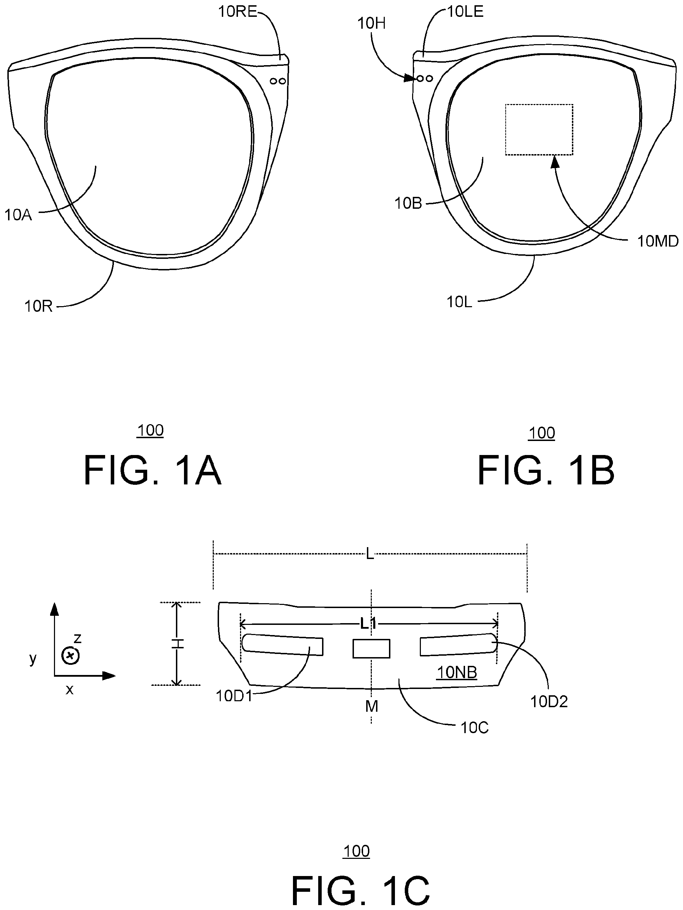

FIG. 1A, FIG. 1B, and FIG. 1C show, for example, some of the interchangeable or locally adjustable components (e.g., right half-frame 10R, left half-frame 10L, and adjustable nose bridge 10NB, respectively) that may be used for assembling the smart eyewear.

As shown in FIG. 1A, right half-frame 10R may hold an optical see-through lens 10A, and as shown in FIG. 1B, left half-frame 10L may hold an optical see-through lens 10B. Lens 10A and lens 10B may be prescription or neutral lenses. One or more of the lenses (e.g., lens 10B) may have an associated virtual display (e.g., virtual display 10MD). Both right half-frame 10R and left half-frame 10L may include one or more screw holes (e.g., screw holes 10H) in respective rim edges (e.g., rim edges 10RE and 10LE) as part of bridge-to-frame fastener arrangements for attachment of the locally adjustable nose bridge (e.g., nose bridge 10NB). For visual clarity, temple pieces that may be attached to right half-frame 10R and left half-frame 10L are not shown in either FIG. 1A or FIG. 1B (and are also not shown in several of the following FIGS.).

A nose bridge may be adapted to use one or more bridge-to-frame fastener arrangements to interconnect right half-frame 10R and a left half-frame 10L to form an eyeglasses frame.

FIG. 1C shows a front view of an example locally adjustable nose bridge (e.g., adjustable nose bridge 10NB) that can be used to interconnect right half-frame 10R and a left half-frame 10L. In example implementations, adjustable nose bridge 10NB may include an horizontal strip of material (e.g., strip 10C) as part of the bridge-to-frame fastener arrangements. Strip 10C may have a generally rectangular shape with a length L along a horizontal axis x and a height H along a vertical axis y. Strip 10C / adjustable nose bridge 10NB may made of material that is bendable about a middle axis M, for example, in the z direction (perpendicular to horizontal axis x and vertical axis y). The bendable material may be an elastic metal, metal alloy, a plastic, a resin, a polymer or a metal-polymer composite.

Strip 10C may include one or more longitudinal or horizontal openings (e.g., slot 10D1, slot 10D2, extending, e.g., from about a middle M of the strip, up to or nearly up to, the left and right side edges of the strip, respectively) as part of the bridge-to-frame fastener arrangements for attachment of adjustable nose bridge 10NB to right half-frame 10R (FIG. 1A) and left half-frame 10L (FIG. 1B). The longitudinal or horizontal openings (e.g., slot 10D1, slot 10D2) may be adapted to receive screws matching screw holes 10H in the respective rim edges of the two half-frames 10R and 10L. In an example implementation, the openings may be formed, for example, by one or more horizontal slots cut in the strip of material (e.g., strip 10C). In the example shown in FIG. 1, the openings may be provided by narrow horizontal slots 10D1 and 10D2 that together or in combination extend, for example, in length L1 from near one edge of strip 10C to near an opposite edge of strip 10C.

Screws passing through the longitudinal or horizontal openings (e.g., narrow horizontal slots 10D1 and 10D2) may be used to affix adjustable nose bridge 10NB to right half-frame 10R and left half-frame 10L. The screws may be used at different positions along the length of narrow horizontal slots 10D1 and 10D2 (e.g., along the x axis) to set different inter-frame separation distances between right half-frame 10R and left half-frame 10L.

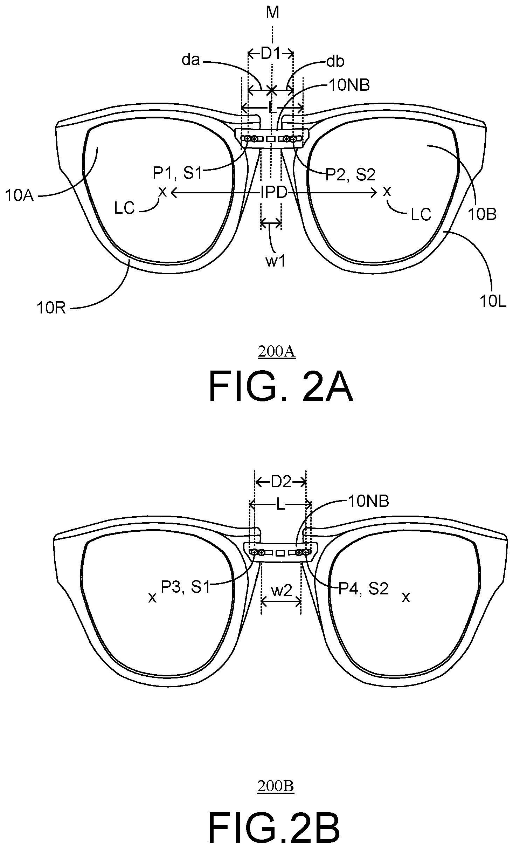

FIG. 2A and FIG. 2B show, for example, eyeglasses frames 200A and 200B with different inter-frame separation distance settings. Eyeglasses frames 200A and 200B may be assembled by using adjustable nose bridge 10NB to interconnect right half-frame 10R and left half-frame 10L. FIG. 2A shows, for example, eyeglasses frame 200A in which screw S1 at position P1 affixes adjustable nose bridge 10NB to right half-frame 10R, and screw S2 at position P2 affixes adjustable nose bridge 10NB to left half-frame 10L. Positions P1 and P2 may be separated by a horizontal distance D1, which is less than the combined horizontal length L1 of horizontal slots 10D1 and 10D2. The horizontal distance D1 (between positions P1 and P2) may correspond to an inter-frame separation distance w1. The inter-frame separation distance w1 may be proportional to an interpupillary distance IPD for eyeglasses frame 200A. An interpupillary distance (IPD) between the person's eye pupils (not shown) may match or correspond to a distance between a middle or center (LC) of lens 10A and a middle or center (LC) of lens 10B.

In example implementations, positions P1 and P2 may be located symmetrically with respect to middle axis M. Thus, inter-frame separation distance w1 may include symmetric or equal contributions (e.g., da, db, da=db)) from the distance of right half-frame 10R from middle axis M and the distance of right half-frame 10R from middle axis M.

In other implementations, positions P1 and P2 may be located asymmetrically with respect to middle axis M (i.e., at unequal distances from middle axis M). Thus, inter-frame separation distance w1 may include asymmetric or unequal contributions (e.g., da, db, da ≠ db) from the distance of right half-frame 10R from middle axis M and the distance of right half-frame 10R from middle axis M.

FIG. 2B shows an example in which the affixing screws (i.e., screw S 1 and screw S2) are used to set a different inter-frame separation distance (e.g., distance w2) between right half-frame 10R and left half-frame 10L in eyeglasses frame 200B than the inter-frame separation distance (e.g., distance w1) of eyeglasses frame 200A shown in FIG. 2A.

FIG. 2B shows, for example, that in eyeglasses frame 200B, screw S 1 at position P3 affixes adjustable nose bridge 10NB to right half-frame 10R, and screw S2 at position P4 affixes adjustable nose bridge 10NB to left half-frame 10L. Positions P3 and P4 may be separated by a horizontal distance D2, which is less than the combined horizontal length L1 of horizontal slots 10D1 and 10D2. Affixing screw S 1 at position P3 and affixing screw S2 at position P4 separated by distance D2 may correspond to an inter-frame separation distance w2. In the examples shown in FIGS. 2A and 2B, inter-frame separation distance w1 in eyeglasses frame 200A (FIG. 2A) may, for example, be smaller than inter-frame separation distance w2 in eyeglasses frame 200B (FIG. 2A).

In an example implementation, eyeglasses frame 200B may be obtained by modifying or adjusting adjustable nose bridge 10NB in place (i.e. in situ) in eyeglasses frame 200A to obtain a different inter-frame separation distance. The adjusting may involve loosening one or both of the screws (i.e., screw S 1 at position P1 and screw S2 at position P2, FIG. 2A), sliding the half-frames along slot 10D1 and or slot 10D2 to the different inter-frame separation distance, and re-tightening the screws at the new positions for eyeglasses frame 200B (i.e., screw S1 at position P3 and screw S2 at position P4, FIG. 2B). A simple tool (e.g., a jeweler's screwdriver) may be used to loosen and retighten the screws.

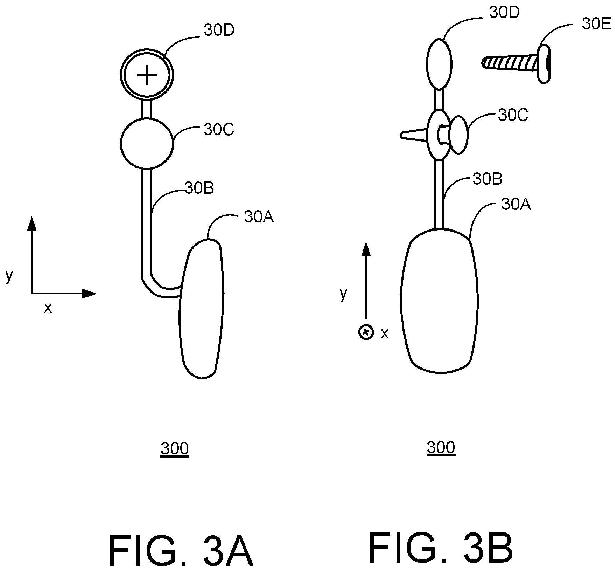

In addition to right half-frame 10R (FIG. 1A), left half-frame 10L (FIG. 1B), and adjustable nose bridge 10NB (FIG. 1C), the interchangeable or locally adjustable components used for assembling the smart eyewear may further include flexible and bendable nose pad assemblies. These nose pad assemblies may be used to set a height of the eyeglasses frame (e.g., frame 200A or frame 200B) when worn by a person. In example implementations, the nose pad assemblies may be adjustable to fit the person's nose and move the eyeglasses frame in three dimensions, for example, to a left, a right, up or down, and in or out, relative to the person's face. FIGS. 3A and 3B respectively show a front view and a side view of an example nose pad assembly 300. Example nose pad assembly 300 may include a nose pad 30A (e.g., a silicone pad) attached to a first end of a generally J-shaped wire 30B. An opposite end (i.e., second end) of J-shaped wire 30B may include a locating pin 30C and a screw-hole guide ring 30D. Screw-hole guide ring 30D may be configured to receive screws (e.g., screw 30E) that may be used to affix nose pad assembly 300 to either right half-frame 10R or left half-frame 10L (or nose bridge 10NB). Locating pin 30C may be used to determine an attachment position of nose pad assembly 300 on rim edges of right half-frame 10R or left half-frame 10L. In the attachment position, screw-hole-guide ring 30D may be aligned with screw holes (not shown) on either the rim edges of right half-frame 10R or left half-frame 10L. These screw holes may be configured for receiving screws (e.g., screw 30E) to affix nose pad assembly 300 to the half-frames. J-shaped wire 30B may be made of bendable material that allows the position of nose pad 30A to be adjusted or moved, for example, to conform to a shape of a person's nose and to set a height of the eyeglasses frame worn by the person.

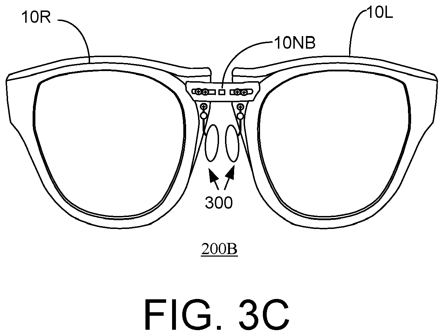

FIG. 3C shows, for example, nose pad assemblies 300 attached to right half-frame 10R and left half-frame 10L in eyeglasses frame 200B (FIG. 2B)

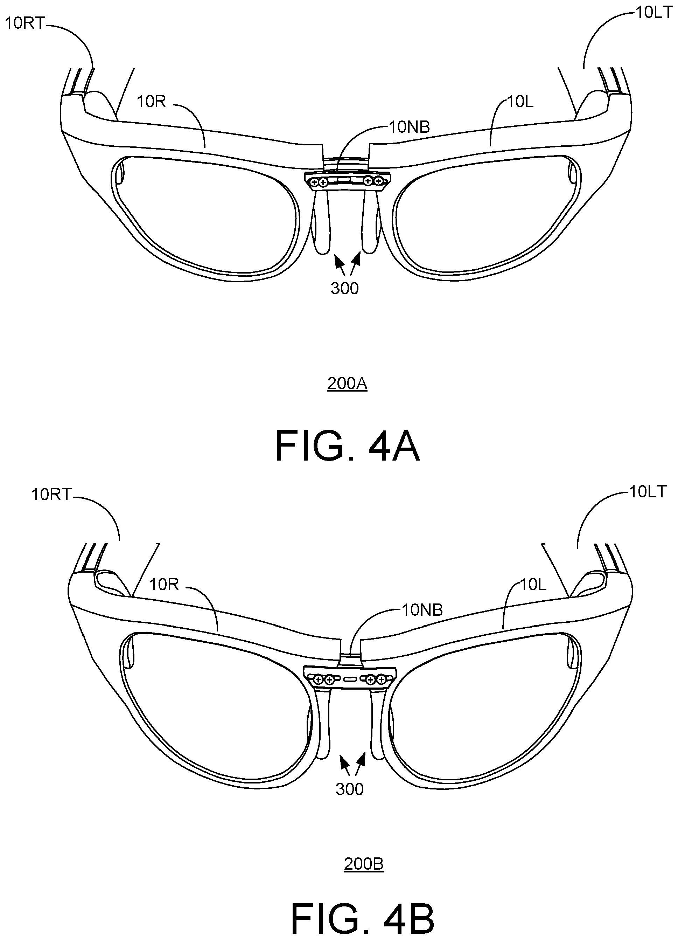

FIGS. 4A and 4B show, for example, perspective views of frames 200A and 200B, respectively, with nose pad assemblies 300 attached to right half-frame 10R and left half-frame 10L.

FIGS. 4A and 4B also show partial views of example temple pieces 10RT and 10LT that may be attached to right half-frame 10R and left half-frame 10L.

In addition to the height of the eyeglasses frame, another geometrical frame parameter that may be important for properly aligning optics of an eyeglasses frame (e.g., frame 200B) to the person's face and eye geometry is a so-called face form angle (also known as bow, wrap angle, or dihedral angle (herein "wrap angle")). The wrap angle may be defined as the angle between the plane of the eyeglasses front and the plane of the right lens shape or the left lens shape.

As noted above, in example implementations, adjustable nose bridge 10NB may be made of material that is bendable, for example, at or about a middle M of strip 10CThe bendable material may be an elastic metal, metal alloy, a plastic, a resin, a polymer or a metal-polymer composite.

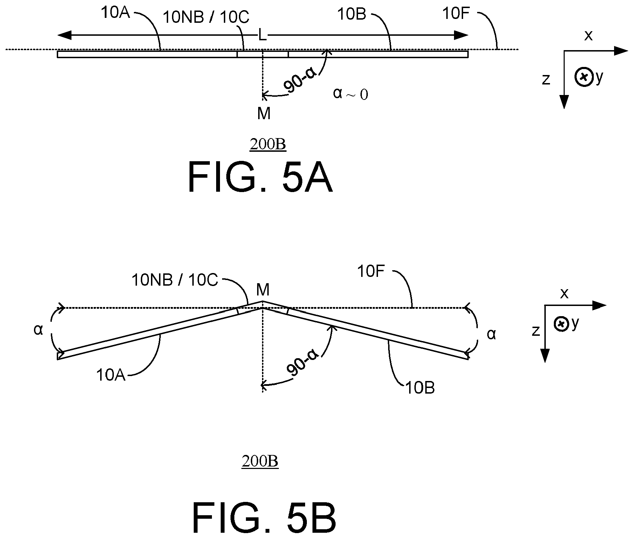

FIG. 5A schematically shows a top view (looking down in the y direction) an eyeglasses frame (e.g., eyeglasses frame 200B) with substantially no wrap (i.e., angle α ~ 0). In the example shown, adjustable nose bridge 10NB / strip 10C is unbent and parallel to the x-y plane (i.e., eyeglasses front 10F). The planes of right lens shape (e.g., lens 10B) and the left lens shape (e.g., lens 10A) are parallel to the x-y plane (i.e., eyeglasses front 10F) with a wrap angle α ~ 0.

FIG. 5B schematically shows a top view (looking down in the y direction) of an eyeglasses frame (e.g., eyeglasses frame 200B) with a non-zero wrap angle α. In the example shown, side portions of adjustable nose bridge 10NB / strip 10C on either side of middle M are bent, for example, in the z direction at about an angle α to the horizontal (i.e., x axis). The plane of right lens shape (e.g., lens 10A) and the plane of left lens shape (e.g., lens 10B) are each at the wrap angle α to the x-y plane (i.e., eyeglasses front 10F).

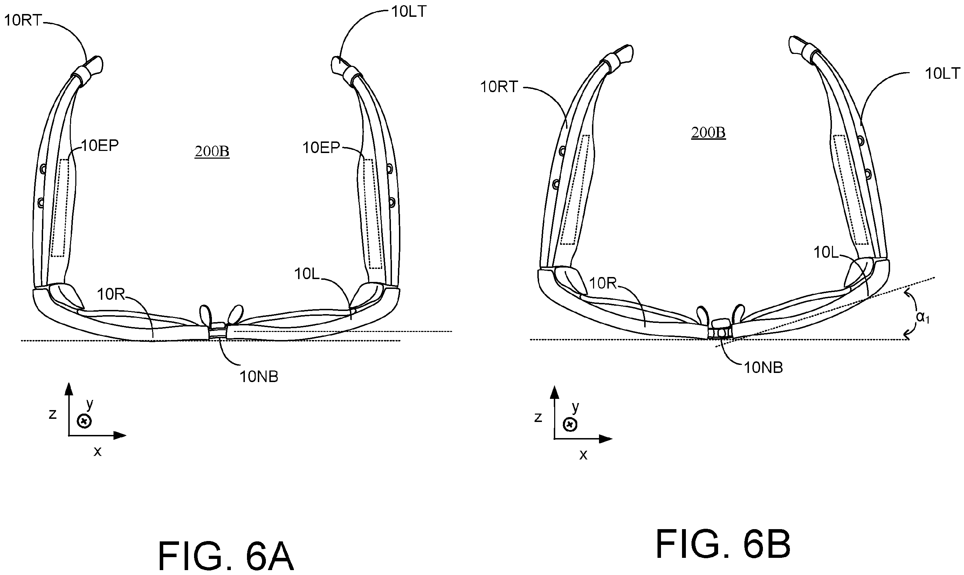

FIG. 6A shows a perspective top view of an eyeglasses frame (e.g., frame 200B) in which right half-frame 10R or left half-frame 10L are interconnected by an adjustable nose bridge (e.g., nose bridge 10NB) with substantially no wrap (i.e., α ~ 0). FIG. 6B shows a perspective top view of a frame (e.g., frame 200B) in which right half-frame 10R and left half-frame 10L are interconnected by an adjustable nose bridge (e.g., nose bridge 10NB) that has been bent to wrap frame 200B with a wrap angle α₁ about the face of a person wearing the frame.

Further, in example implementations, adjustable nose bridge (e.g., nose bridge 10NB) may be configured to adjustably hold each of right half-frame 10R and or left half-frame 10L in a frame (e.g., frame 200B) at an angle with respect to the vertical (x axis) in the x-y plane. When fitting the frame to a person's face and eye geometry, right half-frame 10R and or left half-frame 10L may be rotated angles (e.g., at angles θ₁₁ and θ₂, respectively) with respect to the vertical (x axis) to match or compensate for the cyclo-rotations (e.g., involuntary cyclovergence) of the person's eyes. In example implementations, the cyclo-rotation angle (θ₁ or θ₂,) may be an angle in the range of 0° to 20°.

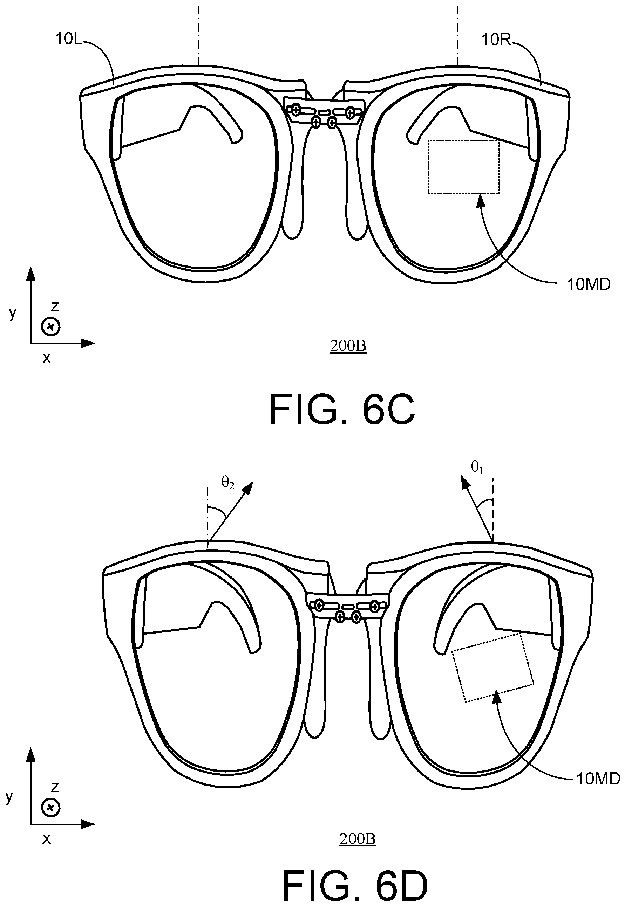

FIG. 6C shows a perspective view of a frame (e.g., frame 200B) in which right half-frame 10R and left half-frame 10L are interconnected by an adjustable nose bridge (e.g., nose bridge 10NB) with substantially no cyclo-rotations. FIG. 6D shows a perspective view of a frame (e.g., frame 200B) in which right half-frame 10R and left half-frame 10L are cyclo-rotated in the x-y plane (e.g., by angles θ1 and θ2, respectively) by rotation relative to the interconnecting adjustable nose bridge (e.g., nose bridge 10NB).

An eyewear (e.g., frame 200A, frame 200B) includes an adjustable nose bridge, two half-frames interconnected by the adjustable nose bridge to form an eyeglasses frame, and a virtual display associated with at least one of the two half-frames. The virtual display having an associated eye box for full viewing of the virtual display. The adjustable nose bridge includes one or more adjustable bridge-to-frame fastener arrangements adapted to provide independent adjustments of one or more geometrical parameters of the eyeglasses frame for aligning a position of the eye box relative to the eyeglasses frame for full viewing of the virtual display. The independent adjustment of each geometrical parameter (e.g., interpupillary distance) of the eyeglasses frame being independent of adjustments of the other geometrical parameters (e.g., wrap angle, cyclo-rotation, tec.) of the eyeglasses frame.

The adjustable nose bridge may thus include one or more adjustable bridge-to-frame fastener arrangements for adjusting one or more geometrical parameters of the eyeglasses frame in order to align a position of the eye box relative to the eyeglasses frame for full viewing of the virtual display.

The one or more adjustable bridge-to-frame fastener arrangements may, for example, allow for adapting one or more of interpupillary distance, nose-to-eye pupil distance, face wrap, head width, and ear position of the eyeglasses frame thereby adjusting at least one of a interpupillary distance parameter, a wrap angle parameter; and a cyclo-rotation parameter of the eyeglasses frame.

A kit for assembling smart eyewear fitted to a person includes two half-frames, at least one of the two half-frames being associated with a virtual display, and a set of interchangeable nose bridges (e.g. nose bridge 10NB, nose bridge 900NB, nose bridge 902NB, nose bridge 1001, nose bridge 1001-1, etc.). Each nose bridge is adapted to couple the two half-frames together as a single frame. The nose bridges in the set of interchangeable nose bridges differ in defining one or more geometrical parameters of the eyeglasses frame with respect to a position of an eye box for viewing the virtual display relative to the eyeglasses frame.

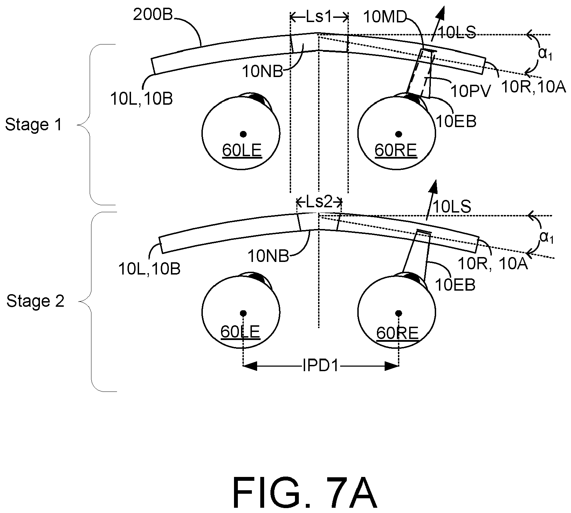

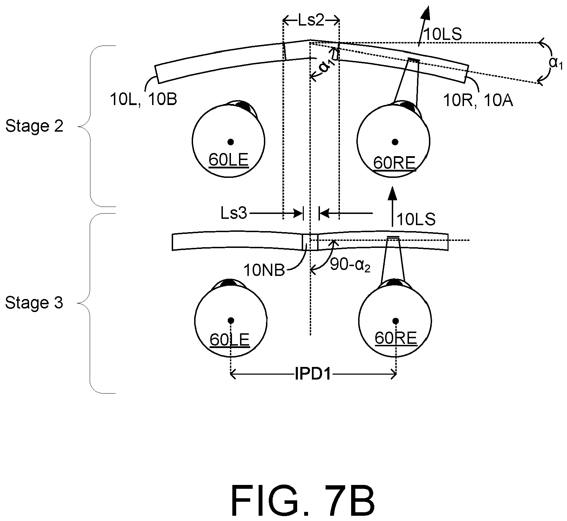

FIGS. 7A and 7B schematically show a fitting procedure for aligning optics of eyeglasses frames (e.g., frame 200B) to the person's face and eye geometry using an adjustable nose bridge (e.g., nose bridge 10NB), in accordance with the principles of the present disclosure.

FIGS. 7A schematically shows an eyeglasses frame (e.g., frame 200B) placed in front a person' eyes during different stages (e.g., stage 1 and stage 2) of the fitting procedure. In the figure, the persons' eyes are represented pictorially as right eye 60RE and left eye 60LE. The persons' eyes may have an interpupillary distance IPD1. A direction of an optical line of sight from the person's right eye 60RE is indicated in the figure by arrow 10LS.

In the fitting procedure, optical see-through lenses 10A and 10B may be placed in front of the person's eyes by eyeglasses frame 200B as worn by the person. In eyeglasses frame 200B, lens 10A and lens 10B may be held by half-frames 10L and 10R that are interconnected by adjustable nose bridge 10NB. Lens 10A and lens 10B may prescription or neutral lenses. Lens 10A may include a virtual display 10MD. Virtual display 10MD may define a volume (i.e., an eye box 10EB, a trapezoidal-shaped frustum volume) in which an eye pupil must be present to have a full view of the micro display. Wrap angle and inter-frame distance adjustments of adjustable nose bridge 10NB may control the orientation and position of the eye box 10EB relative to the person's eye.

At stage 1 of the fitting procedure, adjustable nose bridge 10NB may be set to have an inter-frame separation distance Ls1, and bent to have a wrap angle α₁. At the parameter settings Ls1 and α₁ of frame 200B, as shown in the figure, the pupil of the right eye 60RE may not be fully in eye box 10EB. Inter-frame separation Ls1 may be too wide for the person to have a full view of eye box 10EB/ virtual display 10MD. At the inter-frame separation of Ls1, the person may have only a partial view of virtual display 10MD with a right edge of eye box 10EB/ virtual display 10MD appearing smeared as viewed by right eye 60RE. For purposes of illustration, the person's partial view of virtual display 10MD, at stage 1 of the fitting procedure, is pictorially represented in the figure by dashed lines as a rectangular box 10PV. As shown in the figure, the person's partial view (e.g., rectangular box 10PV) does not fully cover the right side (e.g., side 10EBR) of eye box 10EB. Because of this, the right side of virtual display 10MD may appear to be smeared in the person's view.

To bring virtual display 10MD in full view, the adjustable nose bridge 10NB may need to be adjusted to reduce the inter-frame separation distance. As shown in the figure, at stage 2 of the fitting procedure, adjustable nose bridge 10NB may be adjusted to set the inter-frame separation distance to a value Ls2 (less than Ls1) bringing virtual display10MD in full view. The adjustment of the inter-frame separation distance may be independent of any changes to the wrap angle. As shown in the figure, the wrap angle has an unchanged value of α₁ through stage 1 and stage 2 of the fitting procedure.

While the adjustment of the inter-frame separation distance at stage 2 of the fitting procedure may have brought eye box 10EB/virtual display10MD in full view, as shown in the figure, micro display 10MD may still be positioned to the right in lens 10A so that the person has to look to the right to see the micro display.

Adjustable nose bridge 10NB may be further adjusted to position micro display 10MD in the front and center of lens 10A. As shown in FIG. 7B, at stage 3 of the fitting procedure, micro display10MD/ eye box 10EB may be moved to the front and center, for example, by adjusting adjustable nose bridge 10NB to reduce the inter-frame separation distance and to remove or reduce the wrap angle of frame 200B. FIG. 7B shows, for example, at stage 3 of the fitting procedure, adjustable nose bridge 10NB adjusted to set the inter-frame separation distance to a value Ls3 (less than Ls2), and adjusted to set the wrap angle to a value α₃. These parameter settings (i.e., inter-frame separation distance L3 and wrap angle value α₃ may correspond to placement of micro display10MD/ eye box 10EB directly in front of lens 10A/ right eye 60RE.

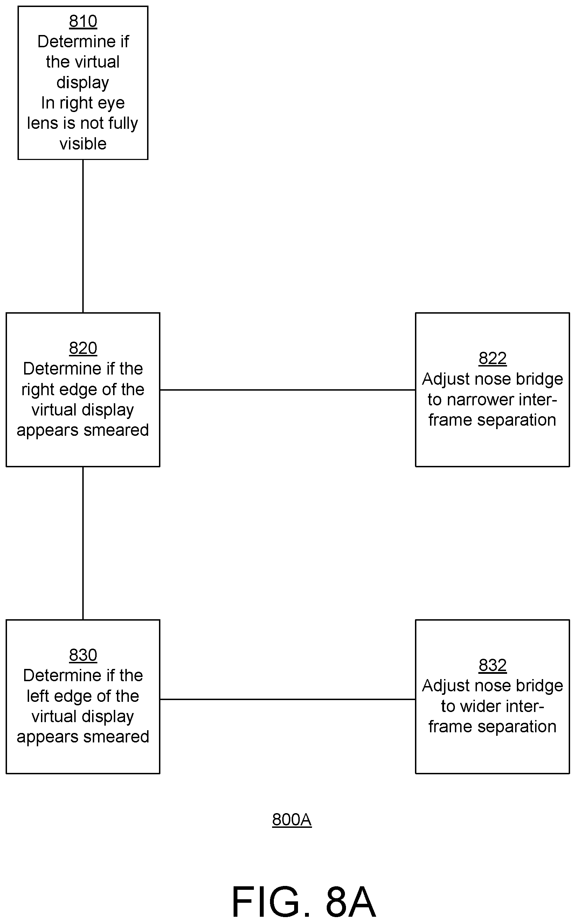

FIG. 8A illustrates an example method 800A for aligning optics of an eyeglasses frame (e.g., frame 200B) to a person's face and eye geometry, in accordance with the principles of the present disclosure. The eyeglasses frame may include a left half-frame and a right half-frame coupled by an adjustable nose bridge (e.g., nose bridge 10NB). The eyeglasses frame may include a virtual display (e.g., embedded in, or overlaid on, a right lens of the eyeglasses), and aligning the optics of the eyeglasses frame may include aligning the position of the micro display in the right lens for full visibility.

Method 800A (with reference to the example shown in FIG. 7A) may include determining that the micro display in the right eye lens of the eyeglasses frame is not fully visible (810). Method 800A may further include determining if a right edge of the micro display appears smeared (820), and adjusting the nose bridge to a narrower inter-frame separation distance setting (822). Method 800A may further include determining if a left edge of the micro display appears smeared (830), and adjusting the nose bridge to a wider inter-frame separation distance setting (832).

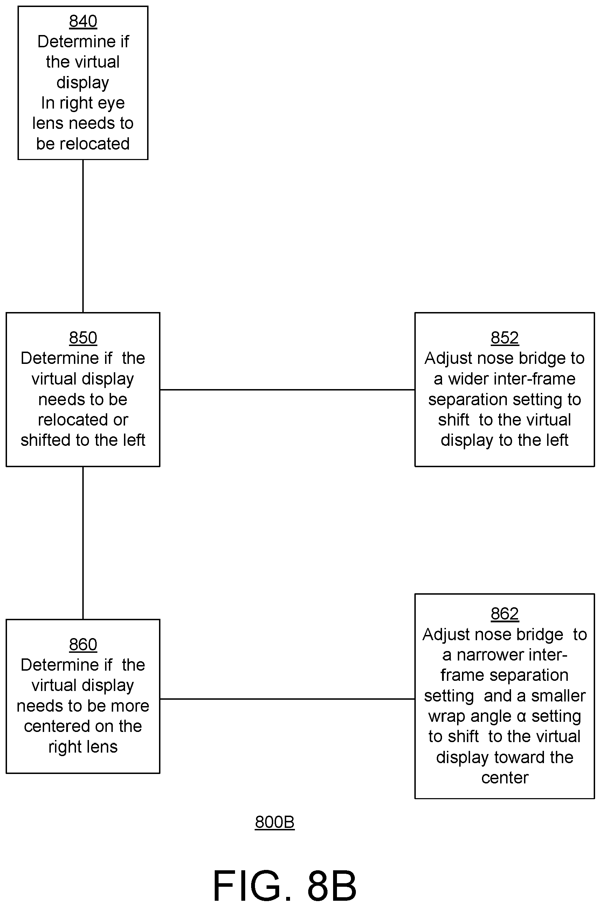

FIG. 8B illustrates an example method 800B for aligning optics of an eyeglasses frame (e.g., frame 200B) to a person's face and eye geometry, in accordance with the principles of the present disclosure. The eyeglasses frame may include a left half-frame and a right half-frame coupled by an adjustable nose bridge (e.g., nose bridge 10NB). The eyeglasses frame may include a virtual display (e.g., associated with a right lens of the eyeglasses), and aligning the optics of the eyeglasses may include aligning the position of the virtual display associated with the right lens to be directly in front and ahead of the person's right eye.

Method 800B (with reference to the example shown in FIG. 7B) may include determining if the micro display in the right eye lens of the eyeglasses needs to be relocated (840). The micro display may need to be relocated, for example, because the present position of micro display may be to the right and not be directly in front and ahead of the person's right eye.

Method 800B may further include determining if the micro display needs to be relocated or shifted to the left (850) and adjusting the nose bridge to a wider inter-frame separation distance setting to shift the micro display to the left (852); and determining if the micro display needs to be more centered on the right lens (860), and adjusting the nose bridge to a narrower inter-frame separation distance setting and a smaller wrap angle α setting to shift to the micro display toward the center (862).

As described previously (e.g., with reference to FIG. 3C), nose assemblies 300 can be attached to directly to right half-frame 10R or left half-frame 10L. In example implementations, nose pad assemblies 300 may be attached to the left and to the right of the nose bridge (e.g., nose bridge 10NB) with clearance for easy nose bridge replacement or adjustment without requiring any additional operations on, or movement of, the nose pads. In addition to the adjustable nose bridge (e.g., nose bridge 10NB), the nose pad assemblies (e.g., nose pad assemblies 300) may be used for aligning optics of eyeglasses frame (e.g., frame 200B) to a person's face and eye geometry. The nose pad assemblies introduce additional degrees of freedom in the individual frame-to-individual person fitting process. Micro-adjustments to the left and to the right nose pads may account for (i.e., mitigate) natural asymmetries of the person's face and nose structures. Further, the nose pad assemblies can be adjusted (e.g., by bending wire 30B, FIG, 3A) to place the eyeglasses frame (e.g., frame 200B) at different positions on the person's nose. The different positions may correspond to placement of micro display 10MD at different heights (e.g., up and down positions) relative to the person's eyes.

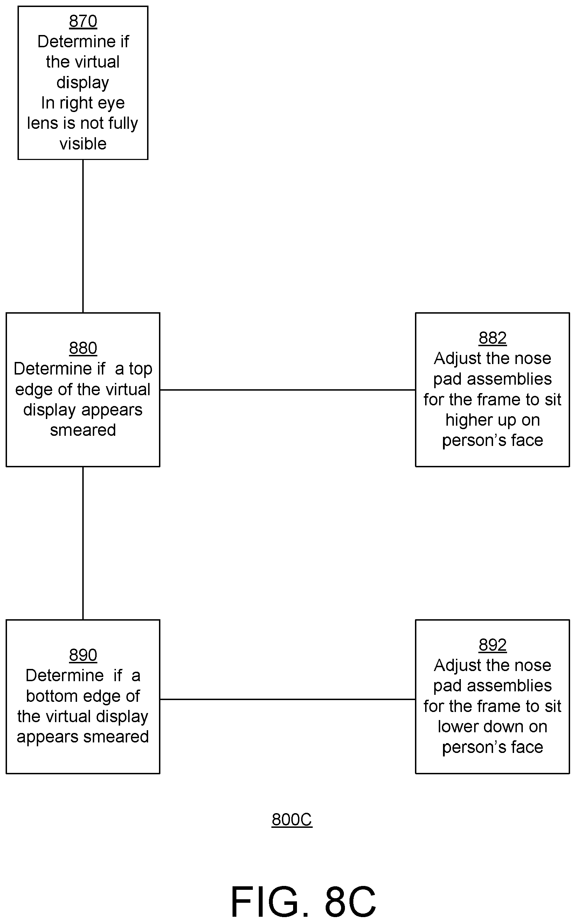

FIG. 8C illustrates an example method 800C for aligning optics of an eyeglasses frame (e.g., frame 200B) to a person's face and eye geometry, in accordance with the principles of the present disclosure. The eyeglasses frame may include a left half-frame and a right half-frame coupled by an adjustable nose bridge (e.g., nose bridge 10NB). The eyeglasses frame may include an associated virtual display (e.g., in a right lens of the eyeglasses), and aligning the optics of the eyeglasses may include aligning the position of the virtual display in the right lens for full visibility. The eyeglasses may include nose pads (e.g., nose pad assemblies 300, FIG. 3A) attached to the left half-frame and right half-frame. The nose pads may mechanically support the frame in front of the person's face and eyes.

Method 800C may include determining that the micro display in the right eye lens of the eyeglasses is not fully visible (870). Method 800A may further include determining if a top edge of the micro display appears smeared (880) and adjusting the nose pad assemblies for the frame to sit higher up on person's face (882); and determining if a bottom edge of the micro display appears smeared (890) and adjusting the nose pad assemblies for the frame to sit lower down on person's face (892).

The flexible and slidable adjustable nose bridges (e.g., nose bridge 10NB) disposed in-between the eyeglasses' lenses can be adjusted during the fitting of the eyeglasses frame for one person, and then readjusted for a different person using simple tools (e.g., a screwdriver). The adjustable nose bridge (e.g., nose bridge 10NB) allows users to continuously change the frame distance to the left eye and the frame distance to the right eye independently. The adjustable nose bridge also allows users to adjust the wrap of the eyeglasses frame around each person's face. Further, nose pad assemblies 300 allow users adjust a height of the eyeglasses frame above the nose. In this manner, a single smart eyeglasses unit assembled with the adjustable nose bridge can be fit to most adult persons using only a simple tool (e.g., a screwdriver). A cosmetic cover can be applied on top of the adjustable nose bridge in the eyeglasses frame.

In other example implementations, a nose bridge having a fixed geometry (i.e., a non-adjustable or a non-sliding geometry) with a predetermined inter-frame separation distance may be used to couple right half-frame 10R and left half-frame 10L to form a fitted eyeglasses frame for a person.

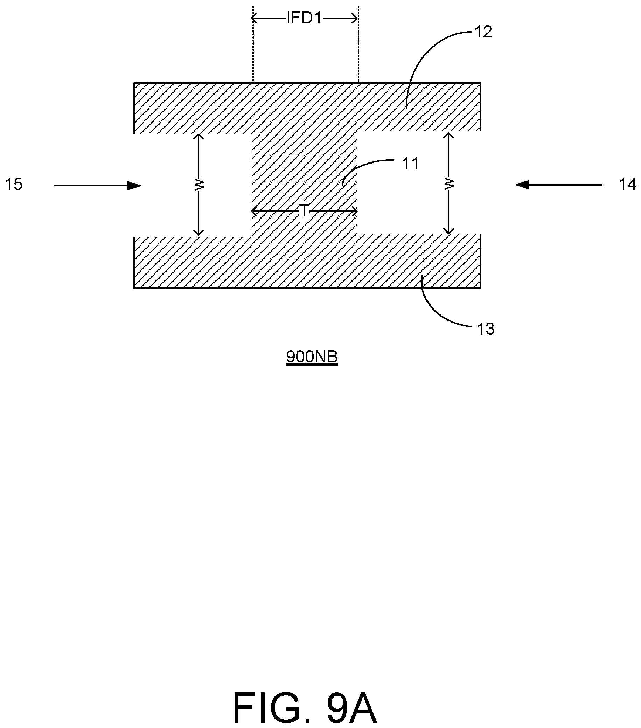

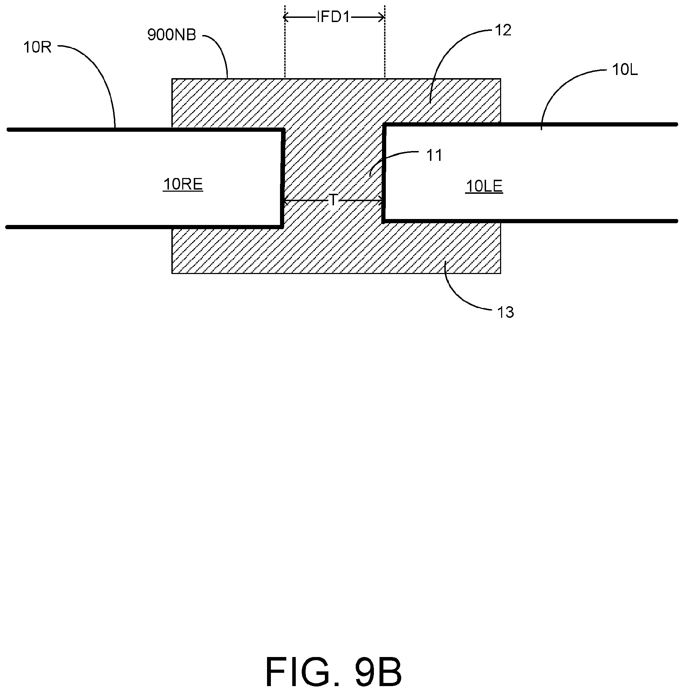

FIG. 9A shows a cross sectional view of an example nose bridge 900NB with a fixed geometry that can be used to couple right half-frame 10R and left half-frame 10L at a predetermined inter-frame separation distance (e.g., predetermined inter-frame separation distance IFD1). Example nose bridge 900NB may have a generally I-beam shape with a central beam or web portion 11 extending between a first flange 12 and a second flange 13. The central beam or web portion 11 may have a thickness T corresponding to the predetermined separation distance IFD1. First flange 12 and the second flange 13 and sidewalls of the central beam or web portion 11 may form generally C-shaped sockets or channels (e.g., channels 14 and 15) on either side of central beam or web portion 11. The C-shaped sockets or channels (e.g., channels 14 and 15) may have opening widths W. The C-shaped sockets or channels 14 and 15 may be configured to receive and hold rim edges of right half-frame 10R and left half-frame 10L, respectively. In example implementations, the opening widths W may, for example, be comparable to the thicknesses of the rim edges (rim edges 10RE, 10LE) of right half-frame 10R and left half-frame 10L. Nose bridge 900NB may be fastened to right half-frame 10R and left half-frame 10L, for example, by an interference fit of the rim edges of the half-frames in the C-shaped sockets or channels 14 and 15. FIG. 9B shows, for example, nose bridge 900NB fastened to right half-frame 10R and left half-frame 10L, for example, by an interference fit of the rim edges (e.g., rim edges 10RE, 10LE) of the half-frames in the C-shaped sockets or channels 14 and 15.

In addition to having a predetermined inter-frame separation distance, a nose bridge with a fixed geometry may be configured to couple right half-frame 10R and left half-frame 10L at a predetermined wrap angle.

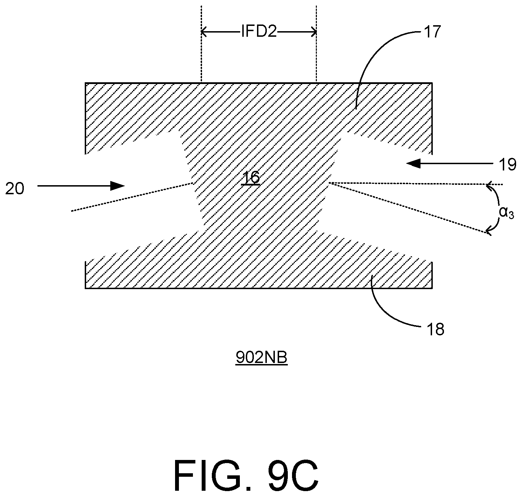

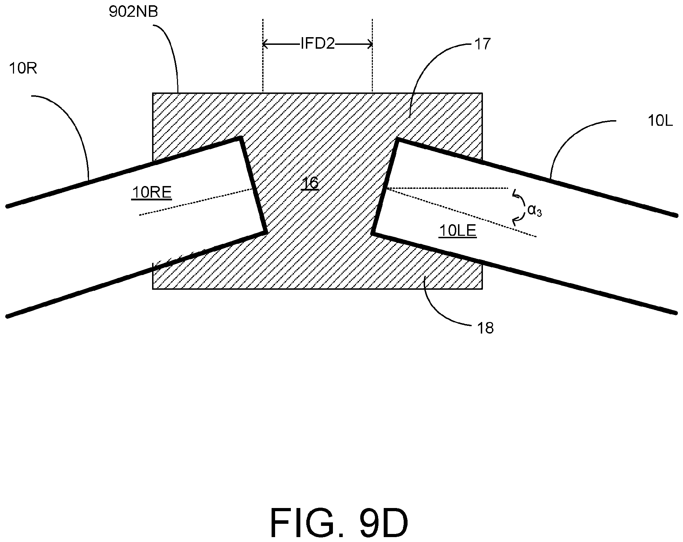

FIG. 9C shows a cross sectional view of an example nose bridge 902NB with a fixed geometry that can be used to couple right half-frame 10R and left half-frame 10L, for example, at a predetermined inter-frame separation distance (e.g., predetermined inter-frame separation IFD2) and a predetermined wrap angle (wrap angle α₃). Example nose bridge 902NB, like example nose bridge 900NB (FIG. 9A), may have a generally I-beam shape with a central beam or web portion 16 extending between a first flange 17 and a second flange 18. The C-shaped sockets or channels 19 and 20 formed by the sidewalls of central beam or web portion 16, first flange 17 and second flange 18, may be configured to receive and hold rim edges of right half-frame 10R and left half-frame 10L, respectively. As shown in FIG. 9D, like nose bridge 900NB, nose bridge 902NB may be fastened to right half-frame 10R and left half-frame 10L, for example, by an interference fit of the rim edges (e.g., rim edge 10RE, rim edge 10LE) of the half-frames in the C-shaped sockets or channels 19 and 20. Right half-frame 10R and left half-frame 10L may be held by nose bridge 902NB at the predetermined separation distance (e.g., predetermined inter-frame separation IFD2) and the predetermined wrap angle (wrap angle α₃) associated with the nose bridge.

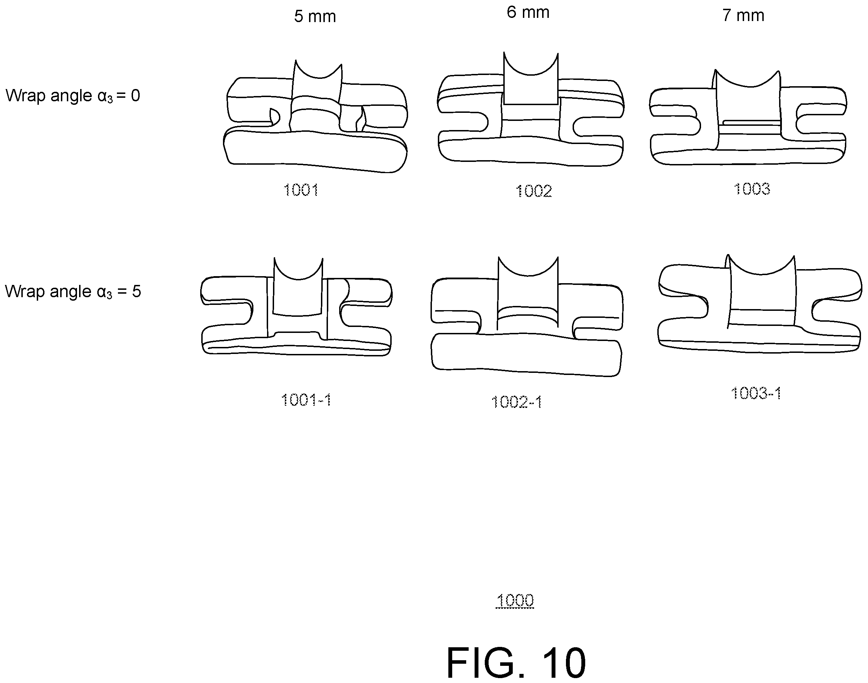

FIG. 10 shows an example eyeglasses component kit (e.g., eyeglasses component kit 1000) for assembling eyeglasses fitted to a person. Glasses component kit 1000 includes a set of interchangeable nose bridges (e.g., nose bridges 1001, 1002, 1003, 1001-1, 1001-2 and 1003-1) with fixed geometry and having predetermined inter-frame separation distances (e.g., like nose bridge 900NB, nose bridge 902NB). Though not explicitly shown in FIG. 10, eyeglasses component kit 1000 may include two half-frames (e.g., a right half-frame 10R and a left half frame10L) each adapted to hold a see-through lens in front of the person's eye. At least one of the see-through lens held by the two half-frames may include a virtual display (e.g., virtual display 10MD). Eyeglasses component kit 1000, may include the set of interchangeable nose bridges in addition to, or as an alternate to, adjustable nose bridge 10NB of kit 100. Each nose bridge may be associated with a respective inter-frame separation distance and a respective wrap angle. The respective inter-frame separation distance and the respective wrap angle of the nose bridge may determine an orientation and a position of a viewing eye box associated with the virtual display relative to the person's eyes.

In an example implementation, eyeglasses component kit 1000 may include a set of interchangeable nose bridges having various combinations of respective predetermined inter-frame separation distance values (e.g., ranging from about 3 mm to 10 mm) and the respective wrap angle values (e.g., ranging from about 0° to 20°). As shown in FIG. 10, the eyeglasses component kit (e.g., kit 1000) may include, for example, a set of six interchangeable nose bridges (e.g., nose bridges 1001, 1002, 1003, 1001-1, 1001-2 and 1003-1). Nose bridge 1001 may have predetermined inter-frame separation distance of 5 mm and predetermined wrap angle 0°; nose bridge 1002 may have predetermined inter-frame separation distance of 6 mm, and predetermined wrap angle 0°; nose bridge 1003 may have predetermined inter-frame separation distance of 7 mm and a predetermined wrap angle of 0°; nose bridge 1001-1 may have predetermined inter-frame separation distance of 5 mm and predetermined wrap angle 5°; nose bridge 1002-1 may have predetermined inter-frame separation distance of 6 mm, and a predetermined wrap angle 5°; nose bridge 1003 may have predetermined inter-frame separation distance of 7 mm, and a predetermined wrap angle of 5°.

In an example procedure for fitting an eyeglasses frame to a person, different nose bridges (e.g., from kit 1000) with different predetermined inter-frame separation and predetermined wrap angle values may be used to couple the two half-frames (e.g., right half-frame 10R and left half-frame 10L) together as a single frame. Appropriate right half-frame 10R and left half-frame 10L may be selected based on a measurement of the person's anthropometric characteristics (e.g., interpupillary distance and head width). Appropriate parameters (e.g., inter-frame separation distance and wrap angle) for aligning optics of the eyeglasses frame to the person's face and eye geometry may be determined, for example, by trial-and-error use of the various nose bridges in kit 1000 to interconnect the half-frames.

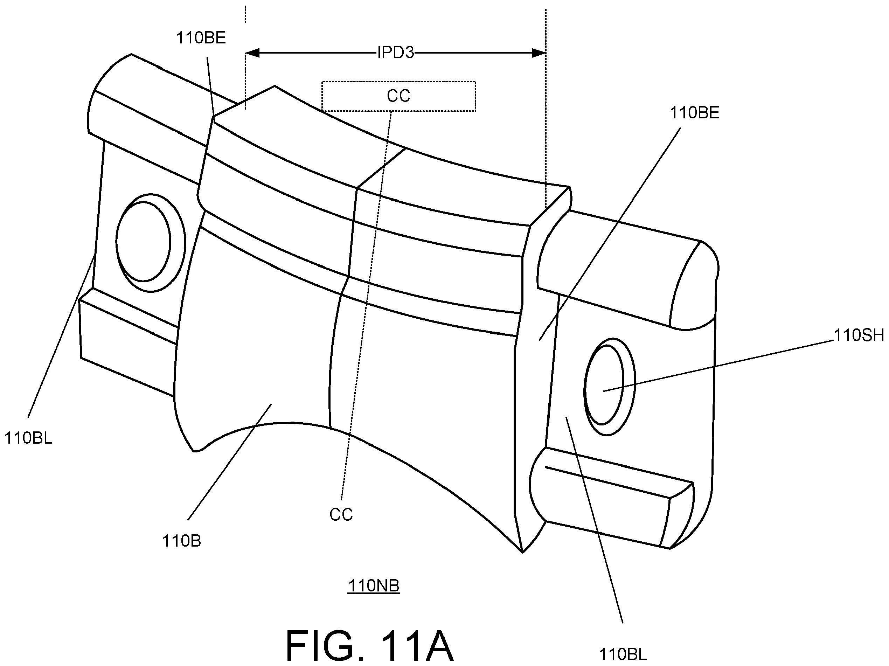

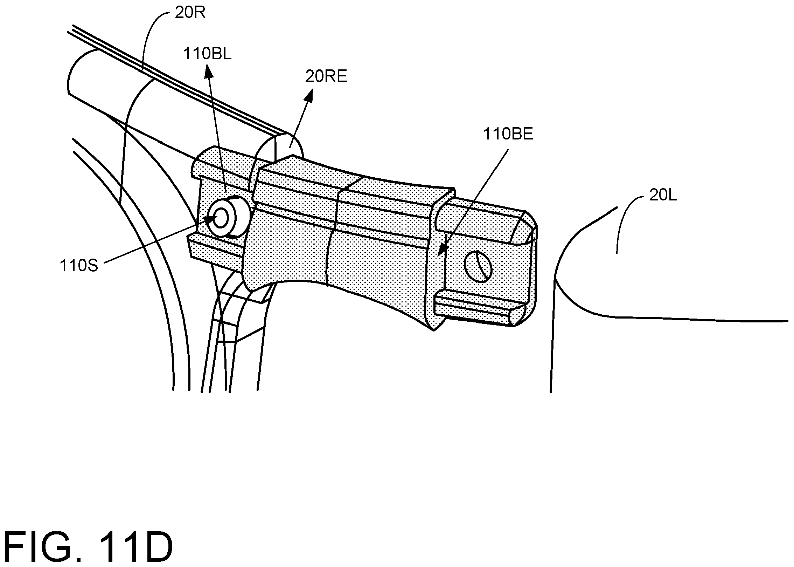

FIG. 11A shows another example nose bridge 110NB having with a fixed geometry that can be used to couple a right half-frame and a left half-frame (e.g., half-frames 20R and 20L, FIGS. 11D-11E) in an eyewear. Nose bridge 110NB may, for example, hold the two half-frames at a predetermined inter-frame separation distance and at a predetermined wrap angle. Example nose bridge 110NB may include a body portion 110B. Body portion 110B may have a generally rectangular block shape having, for example, a length IPD3 between body edge faces 110BE. Legs 110BL may extend generally horizontally (e.g., along the length of body portion 110B) from body edge faces 110BE on a left side and a right side of body portion 110B. Legs 110BL may be inserted (e.g., slid in, pushed in, or clamped in) corresponding holes or slots (e.g., slot 20RH) in edges of the right half-frame and the left half-frame to couple the right half-frame and the left half-frame together. Legs 110BL may, for example, form an interference fit in corresponding holes in the half-frames. Alternatively or additionally, legs 110BL may include screw holes (e.g., screw holes 110SH) for receiving screws to fasten legs 110BL in place in the half-frames. When nose bridge 110NB is deployed to couple the right half-frame and the left half-frame together, body edge faces 110BE may butt against edges of the half-frames and hold the half-frames apart at a distance equal to the length between body edge faces 110BE, i.e., a distance IPD3 apart.

In example implementations, example nose bridge 110NB may be made of bendable material. In addition to having a predetermined inter-frame separation distance, nose bridge 110NB may be bent (e.g., into the plane or out of the plane of the figure on either side of a center axis CC) to couple right half-frame 20R and left half-frame 20L at a predetermined wrap angle (e.g., in a manner similar to nose bridge 902NB as shown in FIG, 9D). In example implementations, legs 110BL may extend out at an angle to the body portion 110B, for example, at an angle to a horizontal along the length of the body portion, the angle being proportional to the respective wrap angle parameter associated with the nose bridge.

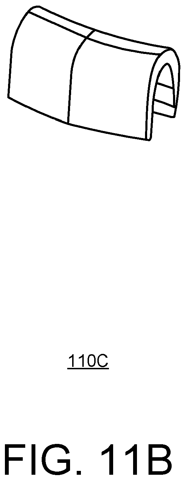

FIG. 11B shows an example cover 110C, that may be placed in an eyeglasses frame assembly over nose bridges (e.g., nose bridge 110NB), for cosmetic or decorative purposes.

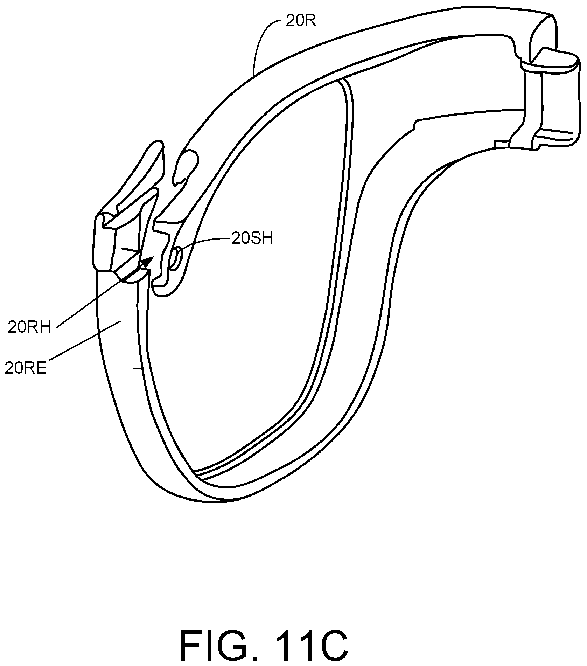

FIG. 11C shows an exploded view of a broken portion of a right half-frame 20R to show, for example, a receiving slot 20RH that can be used to fasten nose bridge 110NB to the right half frame. Receiving slot 20RH may be disposed in an edge 20RE of the right half-frame. Receiving slot 20RH may be configured to receive a leg 1 10BL of nose bridge 110NB for fastening nose bridge 110NB to right half-frame 20R. For example, receiving slot 20RH may have dimensions configured for an interference fit of leg 110BL. Alternatively or additionally, right half-frame 20R may include a screw hole 20SH for receiving a screw to hold leg 110BL in place in receiving slot 20RH.

FIG. 11D illustrates attachment of nose bridge 110NB to right half-frame 20R in a process to couple right half-frame 20R and left half-frame 20L. As shown in the figure, a leg 110BL of nose bridge 110NB is inserted in receiving slot 20RH of right half-frame 20R. A screw 110S (passing through screw holes 20SH and 110SH, not visible) may hold leg 110BL of nose bridge 110NB in place with body portion 110B of nose bridge 110NB held against edge 20RE of the right half frame. In a similar manner, nose bridge 110NB may be held against an edge of the left half frame 20L by inserting a leg 110BL of nose bridge 110NB in a corresponding receiving hole (not shown in FIG. 11D) in the left half-frame.

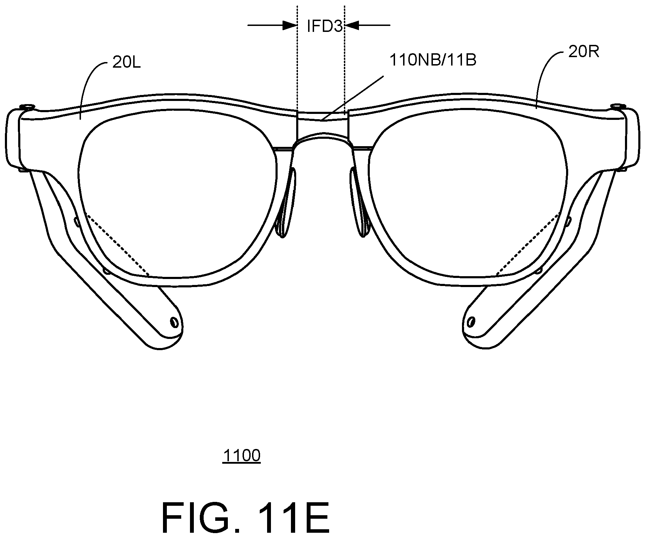

FIG. 11E illustrates eyewear 1100 in which nose bridge 110NB is fastened to right half-frame 20R and left half-frame 20L by inserting and holding legs 110BL of the nose bridge in corresponding receiving holes or slots in the half-frames. (The legs 110BL and the corresponding receiving holes or slots are not visible in the figure). Further, a cover 110C (FIG. 11B) may be placed over nose bridge 110NB for cosmetic purposes, Nose bridge 110NB may hold the two half frames at the predetermined inter-frame separation distance IPD3 and at predetermined wrap angle.

In example implementations, an eyeglasses component kits (e.g., kit 1000, FIG. 10 may include a set of nose bridges (e.g., nose bridge 110NB) with a fixed geometry and having a range of predetermined inter-frame separation distances (e.g., 3 mm, 5 mm, 7 mm, etc.) and a range of predetermined wrap angles (e.g., 0°, 5°, 10°, etc.).

In example implementations, the eyeglasses component kits (e.g., kit 1000) described herein may include covers for the adjustable nose bridges used to interconnect the right half and left half-frames. The covers (e.g., cover 110C, FIG. 11B) may be placed over the adjustable nose bridges, for example, for protective, cosmetic or decorative purposes.

An optometric tool for capturing anthropometric or biometric characteristics of a person may include an eyeglasses component kit (e.g., kit 1000) that can be used to assemble and fit a test eyeglasses frame to a person, in accordance with the principles of the present disclosure. The anthropometric or biometric characteristics may, for example, include interpupillary distance, vertical distance from the nose to the pupils, face wrap, head width, ear position, etc.

The eyeglasses component kit may, for example, include two half-frames that can be interconnected to form the test eyeglasses frame and hold a pair of see-through lenses in front of the person's eyes. A virtual display may be embedded in, or overlaid on, at least one of the pair of see-through lenses.

The eyeglasses component kit may include adjustable or interchangeable nose bridges (e.g., nose bridge 10NB, nose bridge 900NB, nose bridge 902NB, nose bridges 1001, 1002, 1003, 1001-1, 1001-2 and 1003-1, etc.) that can interconnect the two half-frames to form the test eyeglasses frame. The test eyeglasses frame may utilize the nose bridge over the person's nose, and utilize temple pieces that rest over the person's ears to hold the pair of see-through lenses in position in front of the person's eyes. The temple pieces may include electronics to prepare and send stimuli (e.g., captions, images) for display on the virtual display. The electronics in the temple pieces may be coupled to the virtual display (e.g., over wires or wirelessly) and configured to display optical stimuli (e.g., images and patterns) on the virtual display.

The nose bridges may include mechanisms that can provide independent adjustments (i.e., one-by-one adjustments) of geometrical parameters of the test eyeglasses frame (e.g., interpupillary distance, wrap angle, cyclo-rotation, etc.) for aligning optics of the test eyeglasses frame to the person's face and eye geometry.

The eyeglasses component kit may also include a pair of nose pads that can be attached to the two half-frames. The nose pads may be adjustable to make a comfortable fit to the person's nose and to set a height of the test eyeglasses frame on the person's face for aligning optics of the test eyeglasses frame to the person's face and eye geometry.

The optometric tool may be configured (using, e.g., the electronics in the temple pieces) to display test stimuli on the virtual display during the fitting process. The test stimuli may, for example, include an optometric test pattern or image. The optometric test patterns or images, may, for example, include color patterns to assess color uniformity, or line patterns to assess distortion, or dot patterns to assess focus. Multiple test stimuli that capture perception of different qualities may be used.

The optometric tool may record the person's responses to the test stimuli displayed on the virtual display of the test eyeglasses frame, at each stage of the fitting process. The responses may include, for example, one or more of an ability to fully see the micro display (e.g., seeing all four corners of the display; uniformity, brightness, color, sharpness, contrast (and other image characteristics) of the whole image as perceived by the person; uniformity or non-uniformity of focus of the image across the micro display as perceived by the person; vertical and horizontal alignment, keystone and other geometric image distortions as perceived by the person; and other aspects related to image quality and geometry as perceived by the person).

The optometric tool may be provisioned with readout mechanisms to capture data on the test eyeglasses frame configurations (e.g., dimensions, orientations, spacings of the frame components, etc.) at each stage of the fitting process. The readout mechanisms may include encoders/sensors (e.g., mechanical, optical, electrical, sensors etc.). The encoders/sensors may respond to mechanical changes (or a state of the frame configuration) to capture the test eyeglasses frame geometry. Other example readout mechanisms may involve computer vision. For example, camera-based observations may be used to capture a current state of the test eyeglasses frame configuration. In some instances, the readout mechanisms may involve manual measurements and observations (e.g., human observation of distances and angles of the test eyeglasses frame geometry).

A best-fit configuration of the test eyeglasses frame may correspond to a desired or target perception of the stimuli (e.g., a full view of the virtual display) by the person. The optometric tool may capture data on the best-fit test eyeglasses frame configuration (e.g., dimensions, orientations, spacings of the frame components) that corresponds to the desired or target perception of the stimuli.

Data on the test eyeglasses frame configurations (e.g., dimensions, orientations, spacings of the frame components, etc.) and data on the person's responses to various display stimulus presented by the optometric tool during the fitting process may be collected as fitting results data. The fitting results data can be used to determine the geometry of a new glasses frame that matches, for example, the best-fit configuration of the test eyeglasses frame. The new eyeglasses frame may be fabricated as a single piece having a fixed geometry (e.g., a not-modifiable geometry).

The fitting results data obtained by the optometric tool while fitting eyewear may include anthropometric or biometric data that can be used for customization of eyeglasses and also devices other than eyewear for the person. The anthropometric or biometric data may, for example, include one or more of interpupillary distance, wrap angle, cyclo-rotation, etc. The anthropometric data may be used to customize and fit, for example, swimming goggles, or a motorcycle helmet, to the person.

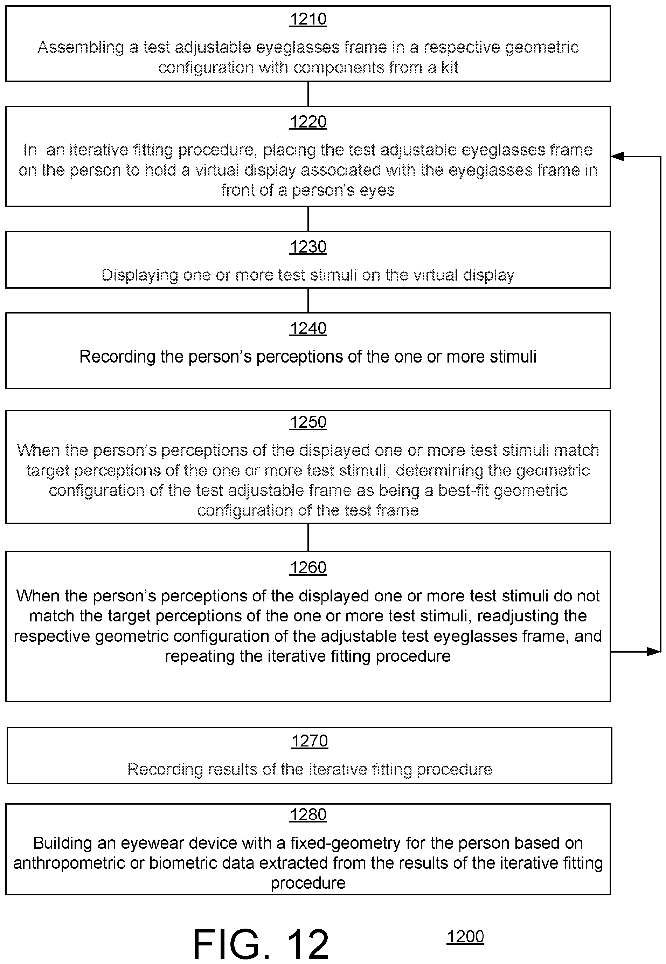

FIG. 12 illustrates a method for capturing anthropometric or biometric characteristics of a person using a glasses component kit (e.g., kit 1000) to assemble and fit a test eyeglasses frame to the person, in accordance with the principles of the present disclosure.

Method 1200 may be implemented using an optometric tool that includes a glasses component kit (e.g., kit 1000) that can be used to assemble and fit a test frame to the person. The test frame may include two half-frames adapted to hold a pair of see-through lenses in front of the person's eyes. A virtual display (e.g., an in-lens micro display) may be embedded in at least one of the pair of see-through lenses, or the virtual display may be a projected display overlaid on at least one of the pair of see-through lenses. The test frame may include electronics (e.g., in the temple pieces) for displaying stimuli (e.g., images and patterns) on the virtual display.

Method 1200 includes assembling a test adjustable eyeglasses frame in a respective geometric configuration with components from the kit (1210).

Method 1200 further includes, in an iterative fitting procedure, placing the test adjustable eyeglasses frame on the person to hold a virtual display associated with the eyeglasses frame in front of a person's eyes in front of the person's eyes (1220), displaying one or more test stimuli on the virtual display (1230), recording the person's perception of the displayed one or more test stimuli (1240).

When the person's perceptions of the displayed one or more test stimuli match target perceptions of the one or more test stimuli, method 1200 includes determining the geometric configuration of the test adjustable frame as being a best-fit geometric configuration of the test frame (1250). When the person's perceptions of the displayed one or more test stimuli do not match the target perceptions of the one or more test stimuli, method 1200 includes readjusting the respective geometric configuration of the adjustable test eyeglasses frame, and repeating the iterative fitting procedure (1260).

Method 1200 further includes recording results of the iterative fitting procedure (1270), and building an eyewear device with a fixed geometry for the person based on anthropometric or biometric data extracted from the results of the iterative fitting procedure (1280).

The adjustable components from the kit used in method 1200 may include components that are adjustable to set one or more of interpupillary distance, vertical distance from the nose to the pupils, face wrap, head width, and ear position of the test adjustable eyeglasses frame. The adjustable components from the kit may include nose bridges and nose pads adjustable to set one or more of interpupillary distance, vertical distance from the nose to the pupils, wrap angles, and cyclo-rotations of the test adjustable eyeglasses frame. Alternatively or additionally, the adjustable components from the kit may include a replaceable nose bridge corresponding to a fixed inter-lens distance and a fixed wrap angle of the test adjustable eyeglasses frame.

In method 1200, displaying one or more test stimuli on the virtual display may include displaying one or more optometric test images and test patterns. The optometric test images and test patterns may include one or more of color patterns to assess color uniformity, or line patterns to assess distortion, or dot patterns to assess focus. Displaying one or more one or more optometric test images and test patterns may include industry-standard optometric test images and test patterns on the virtual display.

In method 1200, the target perceptions of the one or more test stimuli may include one or more of:
seeing a full size of the display;
uniform brightness, color, sharpness, contrast across the display;
uniformity of focus across the display;
vertical and horizontal alignment;
absence of image distortions; and
keystone defect-free image

In method 1200, building an eyewear device with a fixed geometry for the person based on anthropometric or biometric data extracted from the results of the iterative fitting procedure includes building a fixed-geometry eyeglasses frame that matches the best-fit geometric configuration of the test frame.

While example embodiments may include various modifications and alternative forms, embodiments thereof are shown by way of example in the drawings and description herein. It should be understood, however, that there is no intent to limit example embodiments to the particular forms disclosed, but on the contrary, example embodiments are to cover all modifications and alternatives falling within the scope of the claims. Like numbers refer to like elements throughout the description of the figures.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device. Various implementations of the systems and techniques described here can be realized as and/or generally be referred to herein as a circuit, a module, a block, or a system that can combine software and hardware aspects. For example, a module may include the functions/acts/computer program instructions executing on a processor (e.g., a processor formed on a silicon substrate, a GaAs substrate, and the like) or some other programmable data processing apparatus.

Some of the above example embodiments are described as processes or methods depicted as flowcharts. Although the flowcharts describe the operations as sequential processes, many of the operations can be performed in parallel, concurrently or simultaneously. In addition, the order of operations can be re-arranged. The processes can be terminated when their operations are completed, but may also have additional steps not included in the figure. The processes may correspond to methods, functions, procedures, subroutines, subprograms, etc.

Methods discussed above, some of which are illustrated by the flow charts, can be implemented by hardware, software, firmware, middleware, microcode, hardware description languages, or any combination thereof. When implemented in software, firmware, middleware or microcode, the program code or code segments to perform the necessary tasks can be stored in a machine or computer readable medium such as a storage medium. A processor(s) may perform the necessary tasks.

Specific structural and functional details disclosed herein are merely representative for purposes of describing example embodiments. Example embodiments, however, be embodied in many alternate forms and should not be construed as limited to only the embodiments set forth herein.

It will be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing from the scope of the claims. As used herein, the term and/or includes any and all combinations of one or more of the associated listed items.

It will be understood that when an element is referred to as being connected or coupled to another element, it can be directly connected or coupled to the other element or intervening elements can be present. In contrast, when an element is referred to as being directly connected or directly coupled to another element, there are no intervening elements present. Other words used to describe the relationship between elements should be interpreted in a like fashion (e.g., between versus directly between, adjacent versus directly adjacent, etc.).

The terminology used herein is for the purpose of describing particular embodiments only and is not intended to be limiting of example embodiments. As used herein, the singular forms a, an and the are intended to include the plural forms as well, unless the context clearly indicates otherwise. It will be further understood that the terms comprises, comprising, includes and/or including, when used herein, specify the presence of stated features, integers, steps, operations, elements and/or components, but do not preclude the presence or addition of one or more other features, integers, steps, operations, elements, components and/or groups thereof.

It should also be noted that in some alternative implementations, the functions/acts noted may occur out of the order noted in the figures. For example, two figures shown in succession may in fact be executed concurrently or may sometimes be executed in the reverse order, depending upon the functionality/acts involved.

Unless otherwise defined, all terms (including technical and scientific terms) used herein have the same meaning as commonly understood by one of ordinary skill in the art to which example embodiments belong. It will be further understood that terms, e.g., those defined in commonly used dictionaries, should be interpreted as having a meaning that is consistent with their meaning in the context of the relevant art and will not be interpreted in an idealized or overly formal sense unless expressly so defined herein.

Portions of the above example embodiments and corresponding detailed description are presented in terms of software, or algorithms and symbolic representations of operation on data bits within a computer memory. These descriptions and representations are the ones by which those of ordinary skill in the art effectively convey the substance of their work to others of ordinary skill in the art. An algorithm, as the term is used here, and as it is used generally, is conceived to be a self-consistent sequence of steps leading to a desired result. The steps are those requiring physical manipulations of physical quantities. Usually, though not necessarily, these quantities take the form of optical, electrical, or magnetic signals capable of being stored, transferred, combined, compared, and otherwise manipulated. It has proven convenient at times, principally for reasons of common usage, to refer to these signals as bits, values, elements, symbols, characters, terms, numbers, or the like.

In the above illustrative embodiments, reference to acts and symbolic representations of operations (e.g., in the form of flowcharts) that can be implemented as program modules or functional processes include routines, programs, objects, components, data structures, etc., that perform particular tasks or implement particular abstract data types and may be described and/or implemented using existing hardware at existing structural elements. Such existing hardware may include one or more Central Processing Units (CPUs), digital signal processors (DSPs), application-specific-integrated-circuits, field programmable gate arrays (FPGAs) computers or the like.

It should be borne in mind, however, that all of these and similar terms are to be associated with the appropriate physical quantities and are merely convenient labels applied to these quantities. Unless specifically stated otherwise, or as is apparent from the discussion, terms such as processing or computing or calculating or determining of displaying or the like, refer to the action and processes of a computer system, or similar electronic computing device, that manipulates and transforms data represented as physical, electronic quantities within the computer system's registers and memories into other data similarly represented as physical quantities within the computer system memories or registers or other such information storage, transmission or display devices.

Note also that the software implemented aspects of the example embodiments are typically encoded on some form of non-transitory program storage medium or implemented over some type of transmission medium. The program storage medium can be magnetic (e.g., a floppy disk or a hard drive) or optical (e.g., a compact disk read only memory, or CD ROM), and can be read only or random access. Similarly, the transmission medium can be twisted wire pairs, coaxial cable, optical fiber, or some other suitable transmission medium known to the art. The example embodiments not limited by these aspects of any given implementation.

## Claims

1. A method for capturing anthropometric data of a person using an adjustable eyeglasses frame (200A, 200B), the adjustable eyeglasses frame (200A, 200B) holding a pair of lenses (10A, 10B), at least one of the lenses (10A, 10B) associated with a virtual display (10MD), the method comprising:
assembling a test adjustable eyeglasses frame in a respective geometric configuration using adjustable components (10R, 10L, 10NB, 30A), including a right half-frame (10R), a left half-frame (10L), a nose bridge (10NB) and nose pads (30A), from a kit;
in an iterative fitting procedure, placing the test adjustable eyeglasses frame on the person to hold the at least one of the lenses associated with a virtual display in front of the person's eyes (60RE, 60RL);
displaying one or more test stimuli on the virtual display (10MD);
recording the person's perception of the displayed one or more test stimuli;
when the person's perceptions of the displayed one or more test stimuli match target perceptions of the one or more test stimuli, determining the geometric configuration of the test adjustable frame as being a best-fit geometric configuration of the test adjustable eyeglasses frame;
when the person's perceptions of the displayed one or more test stimuli do not match the target perceptions of the one or more test stimuli, readjusting the respective geometric configuration of the adjustable test eyeglasses frame, and repeating the iterative fitting procedure;
recording results of the iterative fitting procedure; and
building an eyewear device having a fixed geometry for the person based on anthropometric data extracted from the results of the iterative fitting procedure.

2. The method of claim 1, wherein the adjustable components (10R, 10L, 10NB, 30A) from the kit include components adjustable to set one or more of interpupillary distance (IPD), nose-to-eye pupil distance, face wrap, head width, and ear position of the test adjustable eyeglasses frame.

3. The method of claim 1 or 2, wherein the nose bridges(10NB) are adjustable to set one or more of interpupillary distance (IPD), wrap angles, and cyclo-rotations of the test adjustable eyeglasses frame.

4. The method of any one of claims 1 to 3, wherein the adjustable components (10R, 10L, 10NB, 30A) from the kit include nose pads (30A) adjustable to set a height of the test adjustable eyeglasses frame when worn by the person.

5. The method of any one of claims 1 to 4, wherein the adjustable components (10R, 10L, 10NB, 30A) from the kit include replaceable fixed-geometry nose bridges (10NB), each replaceable fixed-geometry nose bridge (10NB) corresponding to a fixed inter-frame separation distance and a fixed wrap angle of the test adjustable eyeglasses frame.

6. The method of any one of claims 1 to 5, wherein displaying one or more test stimuli on the virtual display includes displaying one or more of: optometric test images and test patterns including one or more of color patterns to assess color uniformity, line patterns to assess distortion, and dot patterns to assess focus.

7. The method of any one of claims 1 to 6, wherein the target perceptions of the one or more test stimuli include one or more of:
seeing a full size the virtual display;
uniform brightness, color, sharpness, contrast across the virtual display;
uniformity of focus across the virtual display;
vertical and horizontal alignment,
absence of image distortions; and
keystone defect-free image.

8. The method of any one of claims 1 to 7, wherein building an eyewear device with a fixed geometry for the person based on anthropometric data extracted from the results of the iterative fitting procedure includes building a fixed-geometry eyeglasses frame that matches the best-fit geometric configuration of the test adjustable eyeglasses frame, building swimming goggles, or building a motorcycle helmet.

## Patentansprüche

1. Verfahren zum Erfassen anthropometrischer Daten einer Person unter Nutzung eines anpassbaren Brillengestells (200A, 200B), wobei das anpassbare Brillengestell (200A, 200B) ein Paar Gläser (10A, 10B) hält, wobei zumindest eines der Gläser (10A, 10B) mit einer virtuellen Anzeige (10MD) assoziiert ist, das Verfahren umfassend:
Zusammenbauen eines anpassbaren Test-Brillengestells in einer jeweiligen geometrischen Konfiguration unter Nutzung von anpassbaren Komponenten (10R, 10L, 10NB, 30A), einschließlich eines rechten Halbrahmens (10R), eines linken Halbrahmens (10L), eines Nasenstegs (10NB) und Nasenpads (30A), aus einem Bausatz;
Platzieren, in einem iterativen Anpassungsverfahren, des anpassbaren Test-Brillengestells auf der Person, um zumindest eines der mit einer virtuellen Anzeige assoziierten Gläser vor den Augen der Person (60RE, 60RL) zu halten;
Anzeigen von einem oder mehreren Testreizen auf der virtuellen Anzeige (10MD);
Aufzeichnen der Wahrnehmung der angezeigten einen oder mehreren Testreize durch die Person;
wenn die Wahrnehmungen der angezeigten einen oder mehreren Testreize durch die Person mit den Zielwahrnehmungen der einen oder mehreren Testreize übereinstimmen, Ermitteln der geometrischen Konfiguration des anpassbaren Testgestells als eine am besten passende geometrische Konfiguration des anpassbaren Test-Brillengestells;
wenn die Wahrnehmungen der angezeigten einen oder mehreren Testreize durch die Person nicht mit den Zielwahrnehmungen des einen oder der mehreren Testreize übereinstimmen, erneutes Anpassen der jeweiligen geometrischen Konfiguration des anpassbaren Test-Brillengestells und Wiederholen des iterativen Anpassungsverfahrens;
Aufzeichnen der Ergebnisse des iterativen Anpassungsverfahrens; und
Herstellen einer Brillenvorrichtung mit einer festgelegten Geometrie für die Person basierend auf anthropometrischen Daten, die aus den Ergebnissen des iterativen Anpassungsverfahrens extrahiert wurden.

2. Verfahren nach Anspruch 1, wobei die anpassbaren Komponenten (10R, 10L, 10NB, 30A) aus dem Bausatz Komponenten beinhalten, die zum Einstellen von einem oder mehreren von einem Interpupillarabstand (IPD), Nasen-Augen-Pupillenabstand, Gesichtsumfassung, Kopfbreite und Ohrposition des anpassbaren Test-Brillengestells anpassbar sind.

3. Verfahren nach Anspruch 1 oder 2, wobei die Nasenstege (10NB) zum Einstellen von einem oder mehreren des Interpupillarabstandes (IPD), der Umfassungswinkel und der Cyclo-Rotationen des anpassbaren Test-Brillengestells anpassbar sind.

4. Verfahren nach einem der Ansprüche 1 bis 3, wobei die anpassbaren Komponenten (10R, 10L, 10NB, 30A) aus dem Bausatz Nasenpads (30A) beinhalten, die zum Einstellen einer Höhe des anpassbaren Test-Brillengestells beim Tragen durch die Person anpassbar sind.

5. Verfahren nach einem der Ansprüche 1 bis 4, wobei die anpassbaren Komponenten (10R, 10L, 10NB, 30A) aus dem Bausatz austauschbare Nasenstege mit fester Geometrie (10NB) beinhalten, wobei jeder austauschbare Nasensteg mit fester Geometrie (10NB) einem festen Rahmentrennungsabstand und einem festen Umfassungswinkel des anpassbaren Test-Brillengestells entspricht.

6. Verfahren nach einem der Ansprüche 1 bis 5, wobei das Anzeigen eines oder mehrerer Testreize auf der virtuellen Anzeige ein Anzeigen von einem oder mehreren der Folgenden beinhaltet: optometrischen Testbildern und Testmustern einschließlich einem oder mehreren von Farbmustern zur Beurteilung der Farbgleichmäßigkeit, Linienmustern zur Beurteilung der Verzerrung und Punktmustern zur Beurteilung der Schärfe.

7. Verfahren nach einem der Ansprüche 1 bis 6, wobei die Zielwahrnehmungen des einen oder der mehreren Testreize eines oder mehrere beinhaltet von:
Sehen einer virtuellen Anzeige in voller Größe;
einheitliche Helligkeit, Farbe, Schärfe und Kontrast über die gesamte virtuelle Anzeige;
Einheitlichkeit der Schärfe über die gesamte virtuelle Anzeige;
vertikale und horizontale Ausrichtung,
Abwesenheit von Bildverzerrungen; und
Bild ohne Trapezverzerrungen.

8. Verfahren nach einem der Ansprüche 1 bis 7, wobei das Herstellen einer Brillenvorrichtung mit einer festgelegten Geometrie für die Person basierend auf anthropometrischen Daten, die aus den Ergebnissen des iterativen Anpassungsverfahrens extrahiert wurden, das Herstellen eines Brillengestells mit festgelegter Geometrie, das der am besten passenden geometrischen Konfiguration des anpassbaren Test-Brillengestells entspricht, das Herstellen einer Schwimmbrille oder das Herstellen eines Motorradhelms beinhaltet.

## Revendications

1. Procédé de capture de données anthropométriques d'une personne à l'aide d'une monture de lunettes réglable (200A, 200B), la monture de lunettes réglable (200A, 200B) maintenant une paire de verres (10A, 10B), au moins l'un des verres (10A, 10B) étant associé à un afficheur virtuel (10MD), le procédé comprenant :
l'assemblage d'une monture de lunettes réglable d'essai dans une configuration géométrique respective à l'aide de composants réglables (10R, 10L, 10NB, 30A), comportant une demi-monture droite (10R), une demi-monture gauche (10L), un pont nasal (10NB) et des ailettes nasales (30A), provenant d'un kit ;
dans une procédure d'adaptation itérative, le placement de la monture de lunettes réglable d'essai sur la personne pour maintenir l'au moins un des verres associé à un afficheur virtuel devant les yeux de la personne (60RE, 60RL) ;
l'affichage d'un ou de plusieurs stimuli d'essai sur l'afficheur virtuel (10MD) ;
l'enregistrement d'une perception de la personne des un ou plusieurs stimuli d'essai affichés ;
lorsque des perceptions de la personne des un ou plusieurs stimuli d'essai affichés concordent avec des perceptions cibles des un ou plusieurs stimuli d'essai, la détermination de la configuration géométrique de la monture réglable d'essai comme étant une configuration géométrique la mieux adaptée de la monture de lunettes réglable d'essai ;
lorsque des perceptions de la personne des un ou plusieurs stimuli d'essai affichés ne concordent pas avec les perceptions cibles des un ou plusieurs stimuli d'essai, le nouveau réglage de la configuration géométrique respective de la monture de lunettes d'essai réglable, et la répétition de la procédure d'adaptation itérative ;
l'enregistrement des résultats de la procédure d'adaptation itérative ; et
la construction d'un dispositif de lunetterie ayant une géométrie fixe pour la personne sur la base de données anthropométriques extraites des résultats de la procédure d'adaptation itérative.

2. Procédé selon la revendication 1, dans lequel les composants réglables (10R, 10L, 10NB, 30A) provenant du kit comportent des composants réglables pour définir un ou plusieurs parmi une distance interpupillaire (IPD), une distance nez-pupille, un enveloppement facial, une largeur de tête et une position d'oreille de la monture de lunettes réglable d'essai.

3. Procédé selon la revendication 1 ou 2, dans lequel les ponts nasaux (10NB) sont réglables pour définir un ou plusieurs parmi la distance interpupillaire (IPD), des angles d'enveloppement et des cyclorotations2 de la monture de lunettes réglable d'essai.

4. Procédé selon l'une quelconque des revendications 1 à 3, dans lequel les composants réglables (10R, 10L, 10NB, 30A) provenant du kit comportent des ailettes nasales (30A) réglables pour définir une hauteur de la monture de lunettes réglable d'essai lorsqu'elle est portée par la personne.

5. Procédé selon l'une quelconque des revendications 1 à 4, dans lequel les composants réglables (10R, 10L, 10NB, 30A) provenant du kit comportent des ponts nasaux (10NB) à géométrie fixe remplaçables, chaque pont nasal (10NB) à géométrie fixe remplaçable correspondant à une distance de séparation inter-monture fixe et à un angle d'enveloppement fixe de la monture de lunettes réglable d'essai.

6. Procédé selon l'une quelconque des revendications 1 à 5, dans lequel l'affichage d'un ou de plusieurs stimuli d'essai sur l'afficheur virtuel comporte l'affichage d'un ou de plusieurs parmi : des images d'essai et des motifs d'essai optométriques comportant un ou plusieurs parmi des motifs de couleur pour évaluer l'uniformité de couleur, des motifs de ligne pour évaluer la distorsion, et des motifs de points pour évaluer la mise au point.

7. Procédé selon l'une quelconque des revendications 1 à 6, dans lequel les perceptions cibles des un ou plusieurs stimuli d'essai comportent un ou plusieurs parmi :
la visualisation d'une taille complète de l'afficheur virtuel ;
une luminosité, une couleur, une netteté, un contraste uniformes sur tout l'afficheur virtuel ;
une uniformité de mise au point sur tout l'afficheur virtuel ;
l'alignement vertical et horizontal,
l'absence de distorsions d'image ; et
une image exempte de défauts de trapèze.

8. Procédé selon l'une quelconque des revendications 1 à 7, dans lequel la construction d'un dispositif de lunetterie avec une géométrie fixe pour la personne sur la base de données anthropométriques extraites des résultats de la procédure d'adaptation itérative comporte la construction d'une monture de lunettes à géométrie fixe qui concorde avec la configuration géométrique la mieux adaptée de la monture de lunettes réglable d'essai, la construction de lunettes de natation ou la construction d'un casque de motocyclette.
